(19) Europäisches Patentamt
European Patent Office
Office européen des brevets

(11) Numéro de publication : **0 074 304 B1**

(12) # FASCICULE DE BREVET EUROPÉEN

(45) Date de publication du fascicule du brevet : **03.04.85**

(21) Numéro de dépôt : **82401567.1**

(22) Date de dépôt : **24.08.82**

(51) Int. Cl.[4] : **C 07 D313/12**, C 07 D267/18, C 07 D311/80, A 61 K 31/335, A 61 K 31/35, A 61 K 31/55

(54) **Ethers tricycliques, leur préparation et les compositions pharmaceutiques les contenant.**

(30) Priorité : **27.08.81 FR 8116347**

(43) Date de publication de la demande : **16.03.83 Bulletin 83/11**

(45) Mention de la délivrance du brevet : **03.04.85 Bulletin 85/14**

(84) Etats contractants désignés : **AT BE CH DE FR GB IT LI LU NL SE**

(56) Documents cités :
**FR-A- 1 513 909**
**FR-A- 1 518 226**
**FR-A- 2 492 378**
**CHEMICAL ABSTRACTS, vol. 62, 1965, col. 14707d, Columbus Ohio (USA);**
**COLLECTION OF CZECHOSLOVAK CHEMICAL COMMUNICATIONS, vol. 30, 1965, pages 463-471, Prague (CZ); J.O. JILEK et al.: "Neurotrope und Psychotrope Substanzen III. Derivate des Dibenz (b,f) (1,4) Oxazepins"**
**BURGERS MEDICINAL CHEMISTRY 4ème édition, Part III, p. 1022**

(73) Titulaire : **ADIR**
**22, rue Garnier**
**F-92200 Neuilly-sur-Seine (FR)**

(72) Inventeur : **Malen, Charles**
**3 allée Traversière**
**F-94260 Fresnes (FR)**
Inventeur : **Poignant, Jean-Claude**
**rue de la Fontaine St-Mathieu**
**F-91440 Bures S/Yvette (FR)**

**EP 0 074 304 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet de nouveaux éthers tricycliques portant une chaîne aminoalkyle, leur préparation et les compositions pharmaceutiques les contenant.

On connaît des éthers tricycliques portant une chaîne éthylamino alkyle et notamment des dibenzoxazépines psychostimulantes et anti-histaminiques (Burger's Medicinal Chemistry, 4[e] édition, Part. III, p. 1022) ou sédatives (Collection of Czechoslowack Chemical Communications 1965, *30,* 463-471) ou des dibenzoxépines sédatives (FR-A-1 513 909 ; FR-A-1 518 226).

Spécifiquement, l'invention se rapporte à des composés répondant à la formule générale

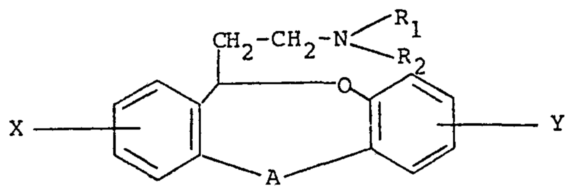

dans laquelle

X et Y identiques ou différents, représentent chacun un atome d'hydrogène ou d'halogène, un groupe alkyle inférieur, alcoxy inférieur ou trifluorométhyle,

A représente un groupe méthylène, ou un groupe imino —$NR_3$— (dans lequel $R_3$ est un atome d'hydrogène, un groupe alkyle inférieur ou un groupe alcanoyle inférieur), et

$R_1$ et $R_2$, identiques ou différents, représentent chacun un atome d'hydrogène ou un groupe alkyle inférieur, ou bien forment ensemble, avec l'atome d'azote auxquels ils sont rattachés, un groupe pyrrolidino, pipéridino ou méthyl-4 pipérazino.

On entend par groupes alkyle, alcoxy ou alcanoyle inférieurs des groupes ayant de 1 à 4 atomes de carbone.

L'invention concerne aussi les sels d'addition des composés de formule générale I avec un acide minéral ou organique thérapeutiquement compatible.

Les composés de formule générale I possèdent un atome de carbone asymétrique et de ce fait peuvent être dédoublés en leurs isomères optiques. Les isomères font partie de l'invention au même titre que les composés racémiques.

Afin de faciliter l'identification des composés des exemples figurant dans le tableau, on peut distinguer les sous-familles de composés suivants :

a) les 6,11-dihydro dibenzo (b,e) oxépines (formule I ; A = méthylène)

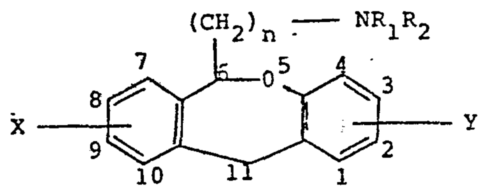

b) les 5,11-dihydro dibenzo (b,e) 1,4-oxazépines (formule I ; A = $NR_3$)

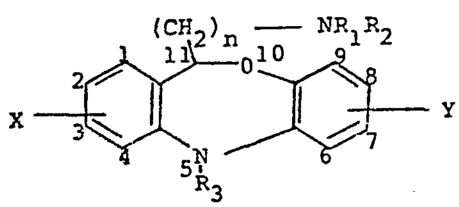

Parmi les acides que l'on peut ajouter aux composés de formule générale I pour former un sel d'addition on pourra citer par exemple, l'acide chlorhydrique, l'acide phosphorique, l'acide tartrique, l'acide citrique, l'acide fumarique, l'acide maléique, l'acide oxalique, etc...

L'invention a aussi pour objet un procédé de préparation des composés de formule générale I, procédé caractérisé en ce que :

a) on soumet une lactone de formule générale (III) de la planche des formules, dans laquelle la signification des substituants X, Y et A est la même que dans la formule (I), à l'action sélective d'un ylide d'alcoxycarbonylméthyl phosphonium de formule (IV)

$$Ar_3P^+—CH^-—COOR_4 \qquad (IV)$$

dans laquelle Ar représente un radical phényle ou phényle substitué et $R_4$ est un radical alkyle inférieur ou aralkyle substitué ou non, pour former le dérivé (alcoxycarbonyl) méthylidénique correspondant de formule générale (V) dans laquelle les substituants A, X, Y et $R_4$ gardent les significations fournies ci-dessus,

b) on soumet celui-ci à l'action d'un agent d'hydrogénation pour former un dérivé (alcoxycarbonyl) méthylénique de formule générale (VI) dans laquelle les substituants X, Y, $R_4$ et A ont les mêmes définitions que ci-dessus, puis

c) ou bien on réduit ce dernier par action d'un agent d'hydrogénation, en particulier d'un hydrure mixte de métal alcalin pour obtenir un alcool de formule générale (VII) de la planche I de formules dans laquelle X, Y et A sont définis comme précédemment,

d) on transforme cet alcool au moyen d'un acide halohydrique ou sulfonique en halogénure ou sulfonate correspondant de formule (VIII) dans laquelle la définition des substituants X, Y et A demeure inchangée et Z est Cl, Br, I, alkyl —$SO_3$ ou aryl —$SO_3$ (en particulier p-toluènesulfonate), puis

e) on fait réagir celui-ci avec une amine de formule générale (IX)

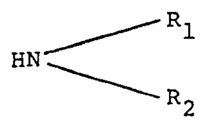

(IX)

dans laquelle $R_1$ et $R_2$ ont la même définition que dans la formule (I) et obtient un dérivé éthylaminé de formule générale (I).

L'invention s'étend également à un autre procédé d'obtention des composés de formule générale (I) caractérisé en ce que :

n) on soumet une lactone de formule générale (III) dans laquelle X, Y et A sont définis comme dans la formule (I) à l'action d'un ylide de cyanométhyl-phosphonium de formule générale (IV')

$$Ar_3P^+—CH^-—CN \qquad (IV')$$

dans laquelle Ar représente un radical phényle éventuellement substitué, pour former un dérivé cyanométhyl idénique de formule générale XIV dans laquelle X, Y et A sont définis comme précédemment (voir planche 2)

o) on hydrogène sélectivement en dérivés amino éthylidéniques correspondants de formule générale XV, sous forme Z ou E dans laquelle les substituants A, X et Y sont définis comme ci-dessus, puis

p) on réduit ces derniers à l'aide d'un agent d'hydrogénation tel qu'un hydrure métallique ou hydrogénation catalytique pour former un dérivé éthylaminé de formule (I) dans laquelle $R_1$ et $R_2$ = H que l'on peut, si on le désire, alkyler (h) avec un agent $R_1Z$ et/ou $R_2Z$ pour obtenir les composés correspondants dans lesquels $R_1$ et/ou $R_2$ est un groupe alkyle inférieur ; Z est un halogène ou un sulfonyle.

L'invention a encore pour objet un procédé d'obtention des composés de formule générale (I)

q) procédé qui consiste à soumettre un composé alcoxycarbonylméthylidénique de formule générale (V) à une saponification ménagée en milieu alcalin pour former un dérivé (hydroxycarbonyl)-méthylidénique de formule générale (XVI) dans laquelle les substituants X, Y et A, ont les significations fournies antérieurement, sous forme Z ou E (voir planche 3),

r) que l'on condense avec une amine de formule générale (IX) définie précédemment, en présence d'un agent de fonctionnalisation du carboxyle (chlorure de cyanuryle ou carbodiimide) pour former l'amide méthylidénique correspondant de formule générale (XVI) dans laquelle les substituants $R_1$, $R_2$, X, Y et A, ont les significations fournies antérieurement,

s) que l'on réduit en dérivé éthylaminé de formule générale (I).

Les composés de formule (I) obtenus selon les procédés décrits ci-dessus peuvent être dédoublés en leurs isomères optiquement actifs par salification à l'aide d'un acide chiral. Ils peuvent aussi être salifiés de manière connue par addition d'un acide minéral ou organique.

Les lactones de départ de formule (III) sont généralement décrites dans la littérature. Toutefois, elles peuvent être obtenues de manière connue par exemple à partir de l'acide benzoïque correspondant de formule générale (II) :

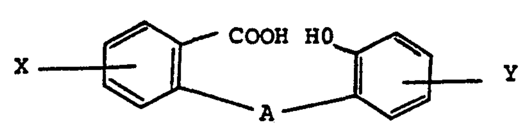

(II)

dans laquelle les substituants X, Y et A ont les mêmes significations que dans la formule (I), que l'on soumet à l'action d'un agent déshydratant tel que l'anhydride acétique ou à l'action de chlorure de thionyle et à la cyclisation par une amine tertiaire pour former la lactone (III).

Les acides benzoïques (II), ainsi que les divers réactifs des formules (IV), (IV') et (IX) sont connues dans la littérature.

Les exemples suivants illustrent l'invention. Les planches de formules jointes explicitent le schéma réactionnel et les différentes structures des composés produits. Pour simplifier la nomenclature, nous convenons de ne pas mentionner le degré d'hydrogénation de l'hétérocycle central.

Exemple 1

dl 6-(2-N-diméthylaminoéthyl)-dibenzo (b,e) oxépine et son fumarate.

a) 6-méthoxycarbonyl-méthylène-dibenzo (b,e) oxépine (formule générale V ; $R_4 = CH_3$).

37,3 g (0,177 mole) de dibenzo (b,e) oxépin-6-one décrite par W. Baker et coll. (J.C.S. 1952, pp. 1452-57), 106,4 g de méthoxycarbonylméthylène triphénylphosphoranne et 375 ml de xylène sont chauffés au reflux pendant 60 heures. Le xylène est éliminé sous vide. Le résidu est repris par 250 $cm^3$ d'éther (oxyde d'éthyle) pour éliminer la triphénylphosphine-oxyde. La phase éthérée est mise à sec. Le résidu (90 g) est partagé de la façon suivante :

diméthylformamide (DMF) 765 ml }
$H_2SO_4$3N 765 ml } et cyclohexane 4 × 900 ml

La phase cyclohexanique est lavée à l'eau et séchée. On évapore à sec. Le résidu (37,5 g) est cristallisé de l'éther isopropylique. On récolte 5,7 g du 1$^{er}$ isomère. F = 164-68°.

Les eaux mères évaporées sont filtrées sur silice et éluées au benzène. On récolte 22,1 g du 2$^e$ isomère.

Rendement (Rt) global (isomères Z + E) = 59 %

RMN : 1$^{er}$ isomère : le signal 1H éthylénique à 5,9 ppm
2$^e$ isomère : le signal 1H éthylénique à 5,3 ppm

Le 2$^e$ isomère est aussi caractérisé par saponification en acide (F = 190-192°).

IR : CO 1 720 $cm^{-1}$ OH 3 100-2 000 $cm^{-1}$
RMN : 8H Ar. (m) 7,1 ppm 1H (s) 5,4 ppm 2H (s) 4 ppm
absence de $OCH_3$

b) 6-méthoxycarbonylméthyl-dibenzo (b,e) oxépine (formule générale VI : $R_4 = CH_3$).

2,7 g de l'ester éthylénique précédent (2$^e$ isomère) sont hydrogénés à pression et température ordinaires dans 30 $cm^3$ d'acide acétique en présence de 0,5 g d'oxyde de platine. On filtre le catalyseur et évapore le solvant sous vide. On obtient après cristallisation dans l'hexane 1,2 g de produit. F = 59-63°.

c) 6-(2-hydroxyéthyl)-dibenzo (b,e) oxépine (formule générale VII).

6,8 g (0,025 mole) de l'ester saturé précédent sont réduits par 52 ml d'une solution de triéthylborohydrure de lithium dans le tétrahydrofuranne THF (0,052 mole). On isole après le traitement habituel et distillation :

1,6 g $E_{0,01}$ = 164-8° F° = 80-81.

d) tosylate de l'alcool précédent (formule VIII ; Z = p-toluènesulfonyle).

2,4 g de l'alcool précédent sont transformés en tosylate par 2 g de p-toluène-sulfochlorure dans la pyridine. On isole 1,5 g de produit. F = 98-102°.

e) dl 6-(2-N-diméthylaminoéthyl)-dibenzo (b,e) oxépine.

3,3 g (0,003 mole) du tosylate précédent sont chauffés en tube scellé à 100° pendant 15 h avec 2,2 g de diméthylamine dans 30 ml d'acétonitrile. On isole, après traitement acide-base, 1,4 g de produit qui sont ensuite transformés en fumarate acide dans l'éthanol.

On obtient : 1,4 g F = 105-125° (déc.).

Exemple 2

dl 11-(2-N-diméthylaminoéthyl) 5-acétyl-dibenzo (b,e) 1,4-oxazépine et son chlorhydrate.

Préparation du produit de départ : 5-acétyl dibenzo (b,e) 1,4-oxazépine-(5H)one (formule générale (III)).

2,1 g (0,01 mole) de dibenzo (b,e) 1,4 oxazépin-(5H)one, préparée selon H. Gurien et coll. (J. Heter. Chem. *3* (4) 527, 1966) sont portés au reflux pendant 2 heures avec 8,5 $cm^3$ d'anhydride acétique. Après évaporation sous vide des produits volatils, le résidu est recristallisé dans 4 $cm^3$ d'acétate d'éthyle. On obtient 1,2 g F = 124-8°.

RMN : 3H singulet à 2,15 ppm 8H (Ar) multiplet 7,4-8 ppm

IR : CO (lactone) 1 730 $cm^{-1}$ CO (acétamide) 1 670 $cm^{-1}$

a) 11-méthoxycarbonylméthylène 5-acétyl-dibenzo (b,e) 1,4-oxazépine (formes Z et E) formule générale V ; $R_4$ = $CH_3$.

30 g (0,12 mole) du dérivé acétylé précédent, 60,2 g (0,18 mole) de méthoxycarbonylméthylène-triphénylphosphoranne et 600 ml de xylène anhydre sont portés au reflux pendant 24 heures. Le solvant est évaporé sous vide. Le résidu brut est chromatographié sur 2 400 g de silice 70-230 mesh (0,063 à 0,20 mm). L'élution est faite par un mélange (50/50) de cyclohexane et d'acétate d'éthyle.

On obtient 30,6 g Rt = 76,2 % F = 108-50°

Un échantillon de chacun des deux isomères est prélevé au cours de la chromatographie et recristallisé dans l'acétonitrile (isomère Z) et l'acétate d'éthyle (isomère E)

RMN : Z 1H (= CH) 5,9 ppm F = 152-57°
E 1H (= CH) 5,5 ppm F = 160-62°

b) dl 11-méthoxycarbonylméthyl 5-acétyl dibenzo (b,e) 1,4-oxazépine (formule générale VI $R_4$ = $CH_3$).

20 g du mélange d'isomères Z + E ci-dessus sont hydrogénés sous pression et à température ordinaires dans 200 ml d'acide acétique en présence de 500 mg d'oxyde de platine. Après absorption de la quantité théorique d'hydrogène, le catalyseur est filtré et le solvant évaporé sous vide. Le résidu est repris par l'éther éthylique, lavé au bicarbonate de sodium 5 %, puis à neutralité. Le solvant est séché et évaporé. Le résidu est recristallisé dans de l'acétate d'éthyle.

On obtient 11,6 g F = 140-44° ; 2e recristallisation 143-5°

IR : CO (ester) 1 725 $cm^{-1}$ CO (amide) 1 660 $cm^{-1}$

RMN : 8H (Ar) (m) 6,7-7,5 ppm 1H (t) 6,1-6,4 ppm
3H (s) 3,8 ppm 2H (t) 3,4 ppm 3H (s) 2,2 ppm

c) dl 11-(2-hydroxyéthyl) 5-acétyl dibenzo (b,e) 1,4-oxazépine. 17 g (0,054 mole) de l'ester méthylique ci-dessus en solution dans 340 ml de THF anhydre sont traités par 108 ml de triéthylborohydrure de lithium N (0,108 mole) dans le THF à température ordinaire pendant 3 h. Le mélange réactionnel est hydrolysé par HCl aqueux dilué, puis le solvant est évaporé sous vide. On extrait au dichlorométhane, lave à la soude diluée puis à l'eau, puis, par évaporation du solvant on obtient 14 g d'un verre constitué par le produit en référence contenant une très faible quantité d'alcool désacétylé (F = 107-109 isolable par chromatographie). L'alcool obtenu n'a pas pu être cristallisé et le produit brut sera donc utilisé dans l'étape suivante.

RMN : 8H Ar (m) 7 ppm 1H 5,8 ppm 2H 3,8 ppm
1H échangeable 2,5 ppm 2H 2,2 ppm 3H (s) 2 ppm

d) p-toluènesulfonate de l'alcool précédent.

27,9 g (0,098 mole) de l'alcool brut ci-dessus sont transformés en ester paratoluènesulfonate par 19 g (0,1 mole) de paratoluène sulfochlorure dans la pyridine. Après le traitement habituel on obtient 30,8 g d'un produit non cristallisable. Rt = 72 %.

RMN : 12H Ar (m) 6,5-8 ppm 1H 5,5-5,9 ppm 2H 4,2-4,5 ppm
6H 2,3 et 2,5 ppm 2H 2-2,2 ppm

e) dl 11-(2-N-diméthylaminoéthyl) 5-acétyl-dibenzo (b,e) 1,4-oxazépine. 11 g (0,025 M) du produit brut obtenu précédemment sont traités par 6,6 g de diméthylamine (0,15 mole) dans 110 ml d'acétonitrile en autoclave à 100° pendant 15 h. Après isolement de la fraction basique on obtient 6,7 g d'une huile qui est transformée en chlorhydrate dans l'éther anhydre. On obtient 6 g de chlorhydrate que l'on recristallise dans l'acétonitrile.

F = 205-225° IR : CO (amide) 1 675 $cm^{-1}$ $NH^+$ 2 400-2 700 $cm^{-1}$

## Exemple 3

dl 11-(2-N-diméthylaminoéthyl)-dibenzo (b,e) 1,4-oxazépine et son chlorhydrate.

0,5 g du composé préparé selon l'exemple 2 dans 1 $cm^3$ de méthanol est traité par 0,5 ml de NaOH à 20 %. On porte au reflux pendant 20 heures. Le solvant est ensuite évaporé sous vide et le résidu extrait par l'éther éthylique est transformé en chlorhydrate. On obtient 0,4 g d'un produit que l'on recristallise.

F = 208-14°

RMN : 8H (Ar) 6,5-7,2 ppm 1H échangeable 6 ppm
1H (t) 4,9-5,2 ppm 10H ($CH_2$) ($CH_3$) 1,9-2,5 ppm

## Exemple 4

dl 11-(2-N-diméthylamino-éthyl) 5-méthyl dibenzo (b,e) 1,4-oxazépine et son fumarate.

3,2 g (0,012 mole) du composé obtenu selon l'exemple 3 sous forme de base sont traités par 4,5 g

(0,12 mole) de $NaBH_4$ dans 42 ml d'acide formique au bain de glace. Après une nuit on dilue à l'eau alcalinisée par l'ammoniaque. Le produit est extrait par le dichlorométhane, lavé puis séché (RMN : $N-CH_3$ à 3,3 ppm). La base est transformée en fumarate acide, recristallisé dans l'éthanol. On obtient 2,5 g.
F = 160-175°

Les composés des exemples précédents ainsi que d'autres composés préparés de la même manière sont rassemblés dans le tableau suivant. On donne la température de fusion (°C) et signale le cas échéant, la décomposition (déc.) et la sublimation (subl.) des composés.
Les composés n$^{os}$ 2 à 4 ont été préparés selon l'exemple 1, les composés 6 et 7 selon l'exemple 2, le composé 10 selon l'exemple 4.

| COMPOSE | X | Y | A | $NR_1$ $R_2$ | Fusion (sel) |
|---|---|---|---|---|---|
| 1 (EX 1) | H | H | $-CH_2-$ | $N(CH_3)_2$ | 105-125° dec. (fumarate) |
| 2 | H | H | $-CH_2-$ | 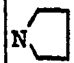 | 154-158° (fumarate) |
| 3 | H | H | $-CH_2-$ | 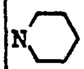 | 226-230° subl. (HCl) |
| 4 | H | H | $-CH_2-$ | 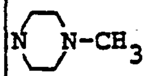 | 215-225° dec. subl (2 HCl) |
| 5 (EX 2) | H | H | $-N(COCH_3)-$ | $N(CH_3)_2$ | 205-225° (HCl) |
| 6 | H | H | $-N(COCH_3)-$ | 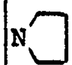 | 220-230° (HCl) |
| 7 | H | H | $-N(COCH_3)-$ | 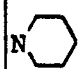 | 245-260° (HCl) |
| 8 (EX 3) | H | H | $-NH-$ | $N(CH_3)_2$ | 208-214° (HCl) |
| 9 (EX 4) | H | H | $-N(CH_3)-$ | $N(CH_3)_2$ | 160-175° (fumarate) |
| 10 | H | H | $-N(C_2H_5)-$ | $N(CH_3)_2$ | 110-116° (fumarate) |

Les composés de l'invention ont été soumis à une étude pharmacologique.

1. Inhibition de l'agressivité

Ce test a été effectué sur des rats et des souris ayant préalablement subi l'ablation des bulbes olfactifs et soumis à l'isolement selon la méthode décrite par L. Valzelli ; Aggressive behaviour *1969*, pp. 70-76 (Excerpa Medica Foundation-Amsterdam).
Aux doses de 10 mg/kg (souris) et 20 mg/kg (rat) par voie intrapéritonéale en solution dans un solvant aqueux, la proportion des souris agressives est diminuée de 40 à 100 % et la proportion des rats muricides de 11 à 55 %. L'effet inhibiteur sur l'agressivité des animaux ne s'accompagne d'aucun effet secondaire tel qu'hyperactivité ou état de dépression.

2. Antagonisme vis-à-vis de la tétrabénazine

Les composés selon l'invention, injectés à des lots de 10 souris aux doses de 10 mg/kg i.p., inhibent de 30 à 100 % la ptôse des paupières provoquée par injection de tétrabénazine. L'activité des composés selon l'invention sur ce test est souvent supérieure à celle de la miansérine prise comme produit de référence.

3. Antagonisme de l'hypothermie

Les composés selon l'invention, injectés par voie i.p. ou s.c. à la dose de 10 mg/kg chez la souris, antagonisent l'hypothermie provoquée par l'injection de 2,5 mg/kg de réserpine s.c. ou de 15 mg/kg s.c. d'apomorphine. Chez certains composés cette activité se manifeste déjà à des doses inférieures à 1 mg/kg.

4. Détermination de la toxicité aiguë

Les composés selon l'invention ont été administrés par voie intrapéritonéale en solution dans l'eau à des doses croissantes s'échelonnant de 20 à 200 mg/kg à des lots de 10 souris de souche Swiss pesant 20 g environ. Les animaux sont gardés 8 jours en observation. On dénombre les morts s'il y en a.

La dose léthale moyenne DL 50 est calculée graphiquement selon la méthode de Tainter et Miller. Elle varie selon les composés entre 80 et 150 mg/kg par voie i.p. et de 250 à 350 mg/kg par voie orale.

5. Examen neurologique

Tous les composés de l'invention ont été administrés à des souris et des rats par voie i.p. à des doses allant de 5 mg/kg à la dose toxique (50 à 150 mg/kg) et ont fait l'objet d'un examen neurologique consistant à observer le comportement des animaux en groupe selon la technique d'Irwin. Pour la plupart des composés aucune modification du comportement des animaux n'était à signaler. A partie des doses de 25 à 50 mg/kg, la motricité, le tonus et les réflexes baissent sensiblement.

Les composés de l'invention sont doués de propriétés pharmacologiques intéressantes. Ce sont des substances psychotropes qui manifestent sur le système nerveux central des effets antidépresseurs et anxiolytiques. Sur l'animal ils entraînent une inhibition de l'agressivité et ont un effet antihypothermique.

Ils sont donc utilisables en thérapeutique humaine ou animale comme médicaments antidépresseurs et/ou anxiolytiques ainsi que dans le traitement des états morbides proches tels que la migraine. Chez l'enfant ils trouvent emploi pour le traitement des difficultés scolaires associées à la dépression.

L'invention a aussi pour objet les compositions pharmaceutiques renfermant au moins un composé de formule générale (I) ou un de ses sels d'addition avec un acide minéral ou organique à titre de principe actif, en association ou en mélange avec un ou plusieurs excipients ou véhicules inertes non toxiques, pharmaceutiquement acceptables.

Parmi les compositions pharmaceutiques selon l'invention on pourra citer plus particulièrement celles qui conviennent pour l'administration par voie parentérale, par voie orale ou par voie rectale et notamment les comprimés nus, enrobés ou à délitement retardé, les gélules, les microcapsules, les pilules, les suspensions, solutions ou émulsions buvables, les gouttes, les solutés ou suspensions injectables conditionnés en ampoules, en flacons multidoses ou en seringues auto-injectables, les suppositoires.

La posologie utile varie selon l'âge et le poids du patient, la voie d'administration et la nature de l'indication thérapeutique.

D'une manière générale la posologie unitaire s'étend de 1 mg à 25 mg et la posologie journalière de 5 à 100 mg chez l'homme. En médecine vétérinaire la posologie sera ajustée au poids de l'animal. Chez l'enfant la posologie pourra être sensiblement diminuée en fonction du poids du sujet.

**Revendications** (pour les Etats contractants : BE, CH, DE, FR, GB, IT, LI, LU, NL, SE)

1. Composés répondant à la formule générale

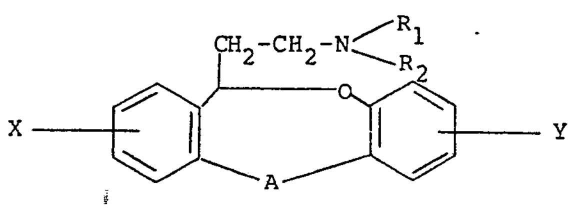

(I)

dans laquelle

X et Y identiques ou différents, représentant un atome d'hydrogène ou d'halogène, un groupe alkyle de 1 à 4 atomes de carbone, alcoxy de 1 à 4 atomes de carbone ou trifluorométhyle,

A représente un groupe méthylène, ou un groupe imino —$NR_3$— dans lequel $R_3$ est un atome d'hydrogène ou groupe alkyle ou alcanoyle de 1 à 4 atomes de carbone, et

$R_1$ et $R_2$ identiques ou différents, représentent chacun un atome d'hydrogène ou un groupe alkyle de 1 à 4 atomes de carbone ou bien forment ensemble, avec l'atome d'azote auxquels ils sont rattachés, un groupe pyrrolidino, pipéridino ou méthyl-4 pipérazino

sous leurs formes racémiques ou d'isomères optiques, ainsi que leurs sels d'addition avec un acide minéral ou organique thérapeutiquement compatible.

2. Composés selon la revendication 1, répondant à la formule générale I dans laquelle A est un groupe méthylène.

3. La 6-(2-N-diméthylaminoéthyl)-dibenzo (b, e) oxépine et son fumarate.

4. La 11-(2-N-diméthylaminoéthyl)-5-méthyl-dibenzo (b, e)-1,4-oxazépine et son fumarate.

5. Les compositions pharmaceutiques renfermant à titre de principe actif au moins un composé selon l'une quelconque des revendications 1 à 4, en association ou en mélange avec un excipient ou un véhicule inerte, non toxique, pharmaceutiquement acceptable.

6. Une composition pharmaceutique selon la revendication 5 dans laquelle l'excipient ou le véhicule est celui adapté pour l'usage par voie parentérale ou par voie orale.

7. Procédé de préparation des composés de formule générale I, selon la revendication 1, procédé caractérisé en ce que

a) on soumet une lactone de formule générale (III) de la planche des formules, dans laquelle la signification des substituants X, Y et A, est la même que dans la formule (I), à l'action sélective d'un ylide d'alcoxycarbonylméthyl) phosphonium de formule (IV)

$$Ar_3P^+—CH^-—COOR_4 \qquad (IV)$$

dans laquelle Ar représente un radical phényle ou phényle substitué et $R_4$ est un radical alkyle inférieur ou aralkyle substitué ou non, pour former le dérivé (alcoxycarbonyl) méthylidénique correspondant de formule générale (V) de la planche 1 dans laquelle les substituants A, X, Y et $R_4$ gardent les significations fournies ci-dessus,

b) on soumet celui-ci à l'action d'un agent d'hydrogénation pour former un dérivé (alcoxycarbonyl) méthylénique de formule générale (VI) de la planche 1 de formules dans laquelle les substituants X, Y, $R_4$ et A ont les mêmes définitions que ci-dessus, puis

c) ou bien on réduit ce dernier par action d'un agent d'hydrogénation, en particulier, d'un hydrure mixte de métal alcalin pour obtenir un alcool de formule générale (VII) de la planche 1 de formules dans laquelle X, Y et A, sont définis comme précédemment,

d) on transforme cet alcool au moyen d'un acide halohydrique ou sulfonique en halogénure ou sulfonate correspondant de formule (VIII) de la planche 1, dans laquelle la définition des substituants X, Y et A demeure inchangée et Z est Cl, Br, I, alkyl-$SO_3$ ou aryl-$SO_3$ (en particulier p-toluènesulfonate), puis

e) on fait réagir celui-ci avec une amine de formule générale (IX)

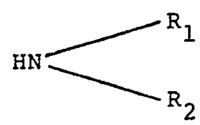

(IX)

dans laquelle $R_1$ et $R_2$ ont la même définition que dans la formule (I) et obtient un dérivé éthylaminé de formule générale (I).

8. Procédé de préparation des composés de formule générale (I) selon la revendication 1, procédé caractérisé en ce que on soumet une lactone de formule générale (III) dans laquelle X, Y et A, sont définis comme dans la formule (I) à l'action d'un ylide de cyanométhyl-phosphonium de formule générale

$$Ar_3P^+—CH^-—CN \qquad (IV')$$

dans laquelle Ar représente un radical phényle éventuellement substitué pour former un dérivé cyanométhylidénique de formule générale XIV de la planche 2, dans laquelle X, Y et A sont définis comme précédemment

o) on hydrogène sélectivement en dérivés amino éthylidéniques correspondant de formule générale XV, sous forme Z ou E, dans laquelle les substituants A, X et Y sont définis comme ci-dessus, puis

p) on réduit ces derniers à l'aide d'un agent d'hydrogénation tel qu'un hydrure métallique ou hydrogénation catalytique pour former un dérivé éthylaminé de formule (I) dans laquelle $R_1$ et $R_2$ = H que l'on peut, si on le désire, alkyler avec $R_1Z$ et/ou $R_2Z$ pour obtenir les composés correspondants, dans lesquels $R_1$ et/ou $R_2$ est un groupe alkyle inférieur (Z est un halogène ou un sulfonyle).

9. Procédé de préparation des composés de formule générale (I) selon la revendication 1, procédé caractérisé en ce que

q) on soumet un composé alcoxycarbonylméthylidénique de formule générale (V) à une saponification ménagée en milieu alcalin pour former un dérivé (hydroxycarbonyl)-méthylidénique de formule générale (XVI) de la planche 3, dans laquelle les substituants X, Y et A, ont les significations fournies antérieurement, sous forme Z ou E,

r) que l'on condense avec une amine de formule générale (IX) définie précédemment, en présence d'un agent de fonctionnalisation du carboxyle (chlorure de cyanuryle ou carbodiimide) pour former

l'amide méthylidénique correspondant de formule générale XVI dans laquelle les substituants $R_1$, $R_2$, X, Y et A, ont les significations fournies antérieurement,

s) que l'on réduit en dérivé éthylaminé de formule générale (I).

**Revendications** (pour l'Etat contractant AT)

1. Procédé de préparation des composés répondant à la formule générale

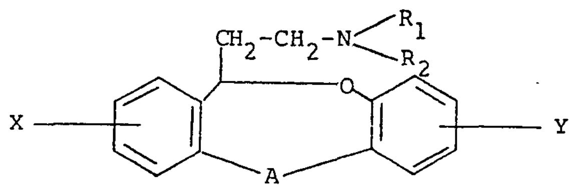

(I)

dans laquelle

X et Y identiques ou différents, représentant un atome d'hydrogène ou d'halogène, un groupe alkyle de 1 à 4 atomes de carbone, alcoxy de 1 à 4 atomes de carbone ou trifluorométhyle,

A représente un groupe méthylène ou un groupe imino —$NR_3$— dans lequel $R_3$ est un atome d'hydrogène ou un groupe alkyle ou alcanoyle de 1 à 4 atomes de carbone, et

$R_1$ et $R_2$ identiques ou différents, représentent chacun un atome d'hydrogène ou un groupe alkyle inférieur, ou bien forment ensemble, avec l'atome d'azote auxquels ils sont rattachés, un groupe pyrrolidino, pipéridino ou méthyl-4 pipérazino

sous leurs formes racémiques ou d'isomères optiques, ainsi que leurs sels d'addition avec un acide minéral ou organique thérapeutiquement compatible

a) on soumet une lactone de formule générale (III) de la planche des formules, dans laquelle la signification des substituants X, Y et A, est la même que dans la formule (I), à l'action sélective d'un ylide d'alcoxycarbonylméthyl) phosphonium de formule (IV)

$$Ar_3P^+—CH^-—COOR_4 \qquad (IV)$$

dans laquelle Ar représente un radical phényle ou phényle substitué et $R_4$ est un radical alkyle inférieur ou aralkyle substitué ou non, pour former le dérivé (alcoxycarbonyl) méthylidénique correspondant de formule générale (V) de la planche 1, dans laquelle les substituants A, X, Y et $R_4$ gardent les significations fournies ci-dessus,

b) on soumet celui-ci à l'action d'un agent d'hydrogénation pour former un dérivé (alcoxycarbonyl) méthylénique de formule générale (VI) de la planche 1 de formules dans laquelle les substituants X, Y, $R_4$ et A ont les mêmes définitions que ci-dessus, puis

c) ou bien on réduit ce dernier par action d'un agent d'hydrogénation, en particulier, d'un hydrure mixte de métal alcalin pour obtenir un alcool de formule générale (VII) de la planche 1 de formules dans laquelle X, Y et A, sont définis comme précédemment,

d) on transforme cet alcool au moyen d'un acide halohydrique ou sulfonique en halogénure ou sulfonate correspondant de formule (VIII) de la planche 1, dans laquelle la définition des substituants X, Y et A demeure inchangée et Z est Cl, Br, I, alkyl-$SO_3$ ou aryl-$SO_3$ (en particulier p-toluènesulfonate), puis

e) on fait réagir celui-ci avec une amine de formule générale (IX)

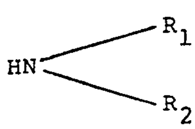

(IX)

dans laquelle $R_1$ et $R_2$ ont la même définition que dans la formule (I) et obtient un dérivé éthylaminé de formule générale (I).

2. Procédé de préparation des composés de formule générale (I) selon la revendication 1, procédé caractérisé en ce que on soumet une lactone de formule générale (III) dans laquelle X, Y et A, sont définis comme dans la formule (I) à l'action d'un ylide de cyanométhyl-phosphonium de formule générale

$$Ar_3P^+—CH^-—CN \qquad (IV')$$

dans laquelle Ar représente un radical phényle éventuellement substitué pour former un dérivé cyanométhylidénique de formule générale XIV de la planche 2, dans laquelle X, Y et A sont définis comme précédemment,

o) on hydrogène sélectivement en dérivés amino éthylidéniques correspondant de formule

générale XV, sous forme Z ou E, dans laquelle les substituants A, X et Y sont définis comme ci-dessus, puis

p) on réduit ces derniers à l'aide d'un agent d'hydrogénation tel qu'un hydrure métallique ou hydrogénation catalytique pour former un dérivé éthylaminé de formule (I) dans laquelle $R_1$ et $R_2$ = H que l'on peut, si on le désire, alkyler avec $R_1Z$ et/ou $R_2Z$ pour obtenir les composés correspondants, dans lesquels $R_1$ et/ou $R_2$ est un groupe alkyle inférieur (Z est un halogène ou un sulfonyle).

3. Procédé de préparation des composés de formule générale (I) selon la revendication 1, procédé caractérisé en ce que

q) on soumet un composé alcoxycarbonylméthylidénique de formule générale (V) à une saponification ménagée en milieu alcalin pour former un dérivé (hydroxycarbonyl)-méthylidénique de formule générale (XVI) de la planche 3, dans laquelle les substituants X, Y et A, ont les significations fournies antérieurement, sous forme Z ou E,

r) que l'on condense avec une amine de formule générale (IX) définie précédemment, en présence d'un agent de fonctionnalisation du carboxyle (chlorure de cyanuryle ou carbodiimide) pour former l'amide méthylidénique correspondant de formule générale XVI dans laquelle les substituants $R_1$, $R_2$, X, Y et A, ont les significations fournies antérieurement,

s) que l'on réduit en dérivé éthylaminé de formule générale (I).

**Claims** (for the Contracting States : BE, CH, DE, FR, GB, IT, LI, LU, NL, SE)

1. Compounds corresponding to the general formula

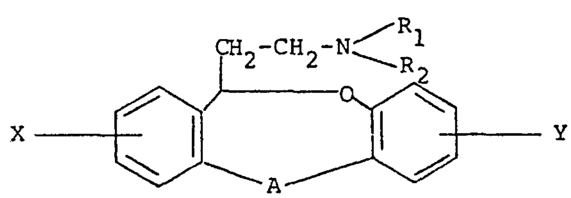

(I)

in which

X and Y are identical or different and represent a hydrogen or halogen atom, an alkyl group having from 1 to 4 carbon atoms, an alkoxy group having from 1 to 4 carbon atoms, or a trifluoromethyl group,

A represents a methylene group or an imino group $NR_3$ in which $R_3$ is a hydrogen atom or an alkyl or alkanoyl group having from 1 to 4 carbon atoms, and

$R_1$ and $R_2$ are identical or different and each represents a hydrogen atom or an alkyl group having from 1 to 4 carbon atoms, or $R_1$ and $R_2$ together form, with the nitrogen atom to which they are bonded, a pyrrolidino, piperidino or 4-methylpiperazino group,

in racemic form or in the form of their optical isomers, and also their addition salts with a therapeutically compatible mineral or organic acid.

2. Compounds according to claim 1, corresponding to the general formula I in which A is a methylene group.

3. 6-(2-N-dimethylaminoethyl)-dibenzo [b,e] oxepin and its fumarate.

4. 11-(2-N-dimethylaminoethyl)-5-methyldibenzo-[b,e]-1,4-oxazepin and its fumarate.

5. Pharmaceutical compositions containing as active ingredient at least one compound according to any one of claims 1 to 4, in admixture or conjunction with a pharmaceutically acceptable non-toxic inert excipient or vehicle.

6. Pharmaceutical composition according to claim 5, in which the excipient or the vehicle is one suitable for parenteral or oral use.

7. Process for the preparation of compounds of the general formula I according to claim 1, characterised in that

a) a lactone of the general formula (III) shown on the sheet of formulae, in which the meanings of the substituents X, Y and A are the same as given in formula (I), is subjected to the selective action of an alkoxycarbonylmethylide phosphonium compound of the formula (IV)

$$Ar_3P^+—CH^-—COOR_4 \qquad (IV)$$

in which Ar represents a phenyl radical or a substituted phenyl radical and $R_4$ is an unsubstituted or substituted lower alkyl or aralkyl radical, to form the corresponding alkoxycarbonylmethylidene derivative of the general formula (V) shown on sheet 1, in which the substituents A, X, Y and $R_4$ have the same meanings as given above,

b) the latter is subjected to the action of a hydrogenation agent to form an alkoxycarbonylmethylene derivative of the general formula (VI) shown on sheet 1 of the formulae in which the substituents X, Y, $R_4$ and A have the same meanings as above, then

c) or the latter is reduced by the action of a hydrogenation agent, especially a mixed alkali metal

hydride, to obtain an alcohol of the general formula (VII) shown on sheet 1 of the formulae in which X, Y and A are defined as above,

d) this alcohol is converted by means of a hydrohalic or sulphonic acid into a corresponding halide or sulphonate of the formula (VIII) shown on sheet 1, in which the definition of the substituents X, Y and A remains unchanged and Z is Cl, Br, I, alkyl-$SO_3$ or aryl-$SO_3$ (especially p-toluenesulphonate), then

e) the latter is reacted with an amine of the general formula (IX)

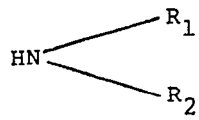

(IX)

in which $R_1$ and $R_2$ have the same definition as in formula (I) and an aminoethyl derivative of the general formula (I) is obtained.

8. Process for the preparation of compounds of the general formula (I) according to claim 1, characterised in that a lactone of the general formula (III), in which X, Y and A are defined as in formula (I), is subjected to the action of a cyanomethylide phosphonium compound of the general formula

$$Ar_3P^+—CH^-—CN \qquad (IV')$$

in which Ar represents an optionally substituted phenyl radical to form a cyanomethylidene derivative of the general formula XIV shown on sheet 2, in which X, Y and A are defined as above,

o) selective hydrogenation is carried out to form corresponding aminoethylidene derivatives of the general formula XV, in Z or E form, in which the substituents A, X and Y are defined as above, then

p) the latter are reduced using a hydrogenation agent such as a metal hydride or using catalytic hydrogenation, to form an aminoethyl derivative of the formula (I) in which $R_1$ and $R_2$ = H, which may, if desired, be alkylated with $R_1Z$ and/or $R_2Z$ to obtain the corresponding compounds in which $R_1$ and/or $R_2$ is a lower alkyl group (Z is a halogen atom or a sulphonyl group).

9. Process for the preparation of compounds of the general formula (I) according to claim 1, characterised in that

q) an alkoxycarbonylmethylidene compound of the general formula (V) is subjected to hydrolysis carried out in an alkaline medium to form a hydroxycarbonylmethylidene derivative of the general formula (XVI) shown on sheet 3, in which the substituents X, Y and A have the meanings given above, in Z or E form,

r) which is condensed with an amine of the general formula (IX) defined above, in the presence of an agent for functionally modifying carboxy (cyanuryl chloride or carbodiimide) to form the corresponding amidomethylidene of the general formula XVII in which the substituents $R_1$, $R_2$, X, Y, and A have the meanings given above,

s) which is reduced to form an aminoethyl derivative of the general formula (I).

**Claims** (for the Contracting State AT)

1. Process for the preparation of compounds corresponding to the general formula

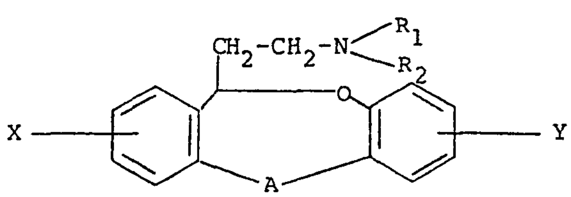

(I)

in which

X and Y are identical or different and represent a hydrogen or halogen atom, an alkyl group having from 1 to 4 carbon atoms, an alkoxy group having from 1 to 4 carbon atoms, or a trifluoromethyl group,

A represents a methylene group or an imino group $NR_3$ in which $R_3$ is a hydrogen atom or an alkyl or alkanoyl group having from 1 to 4 carbon atoms, and

$R_1$ and $R_2$ are identical or different and each represents a hydrogen atom or a lower alkyl group, or $R_1$ and $R_2$ together form, with the nitrogen atom to which they are bonded, a pyrrolidino, piperidino or 4-methylpiperazino group,

in racemic form or in the form of their optical isomers, and also their addition salts with a therapeutically compatible mineral or organic acid, characterised in that

a) a lactone of the general formula (III) shown on the sheet of formulae, in which the meanings of the substituents X, Y and A are the same as given in formula (I), is subjected to the selective action of an

alkoxycarbonylmethylide phosphonium compound of the formula (IV)

$$Ar_3P^+—CH^-—COOR_4 \qquad (IV)$$

in which Ar represents a phenyl radical or a substituted phenyl radical and $R_4$ is an unsubstituted or substituted lower alkyl or aralkyl radical to form the corresponding alkoxycarbonylmethylidene derivative of the general formula (V) shown on sheet 1, in which the substituents A, X, Y and $R_4$ have the same meanings as given above,

b) the latter is subjected to the action of a hydrogenation agent to form an alkoxycarbonylmethylene derivative of the general formula (VI) shown on sheet 1 of the formulae in which the substituents X, Y, $R_4$ and A have the same meanings as above, then

c) or the latter is reduced by the action of a hydrogenation agent, especially a mixed alkali metal hydride, to obtain an alcohol of the general formula (VII) shown on sheet 1 of the formulae in which X, Y and A are defined as above,

d) this alcohol is converted by means of a hydrohalic or sulphonic acid into a corresponding halide or sulphonate of the formula (VIII) shown on sheet 1, in which the definition of the substituents X, Y and A remains unchanged and Z is Cl, Br, I, alkyl-$SO_3$ or aryl-$SO_3$ (especially p-toluenesulphonate), then

e) the latter is reacted with an amine of the general formula (IX)

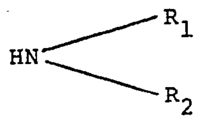

(IX)

in which $R_1$ and $R_2$ have the same definitions as in formula (I) and an aminoethyl derivative of the general formula (I) is obtained.

2. Process for the preparation of compounds of the general formula (I) according to claim 1, characterised in that a lactone of the general formula (III), in which X, Y and A are defined as in formula (I), is subjected to the action of a cyanomethylide phosphonium compound of the general formula

$$Ar_3P^+—CH^-—CN \qquad (IV')$$

in which Ar represents an optionally substituted phenyl radical to form a cyanomethylidene derivative of the general formula XIV shown on sheet 2, in which X, Y and A are defined as above,

o) selective hydrogenation is carried out to form corresponding aminoethylidene derivatives of the general formula XV, in Z or E form, in which the substituents A, X and Y are defined as above, then

p) the latter are reduced using a hydrogenation agent such as a metal hydride or using catalytic hydrogenation, to form an aminoethyl derivative of the formula (I) in which $R_1$ and $R_2$ = H, which may, if desired, be alkylated with $R_1Z$ and/or $R_2Z$ to obtain the corresponding compounds in which $R_1$ and/or $R_2$ is a lower alkyl group (Z is a halogen atom or a sulphonyl group).

3. Process for the preparation of compounds of the general formula (I) according to claim 1, characterised in that

q) an alkoxycarbonylmethylidene compound of the general formula (V) is subjected to hydrolysis carried out in an alkaline medium to form a hydroxycarbonylmethylidene derivative of the general formula (XVI) shown on sheet 3, in which the substituents X, Y and A have the meanings given above, in Z or E form,

r) which is condensed with an amine of the general formula (IX) defined above, in the presence of an agent for functionally modifying carboxy (cyanuryl chloride or carbodiimide) to form the corresponding amidomethylidene of the general formula XVII in which the substituents $R_1$, $R_2$, X, Y, and A have the meanings given above,

s) which is reduced to form an aminoethyl derivative of the general formula (I).

**Ansprüche** (für die Vertragsstaaten : BE, CH, DE, FR, GB, IT, LI, LU, NL, SE)

1. Verbindungen der allgemeinen Formel

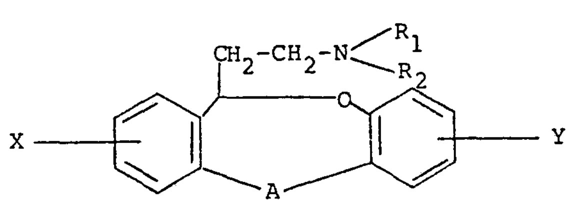

(I)

in der

X und Y, die gleichartig oder verschieden sind, Wasserstoffatome oder Halogenatome, Alkylgruppen mit 1 bis 4 Kohlenstoffatomen, Alkoxygruppen mit 1 bis 4 Kohlenstoffatomen oder Trifluormethylgruppen,

A eine Methylengruppe oder eine Iminogruppe der Formel $—NR_3—$, worin $R_3$ für ein Wasserstoffatom oder eine Alkyl- oder Alkanoyl-Gruppe mit 1 bis 4 Kohlenstoffatomen steht, und

$R_1$ und $R_2$, die gleichartig oder verschieden sein können, jeweils Wasserstoffatome oder Alkylgruppen mit 1 bis 4 Kohlenstoffatomen oder gemeinsam mit dem Stickstoffatom, an das sie gebunden sind, eine Pyrrolidinogruppe, eine Piperidinogruppe oder eine 4-Methyl-piperazinogruppe bedeuten, in Form des Racemats oder der optischen Isomeren sowie deren Additionssalze mit anorganischen oder organischen therapeutisch verträglichen Säuren.

2. Verbindungen nach Anspruch 1 der allgemeinen Formel I in der A für eine Methylengruppe steht.

3. 6-(2-N-Dimethylaminoethyl)-dibenzo(b,e)oxepin und dessen Fumarat.

4. 11-(2-N-Dimethylaminoethyl)-5-methyl-dibenzo(b,e)-1,4-oxazepin und dessen Fumarat.

5. Pharmazeutische Zubereitungen enthaltend als Wirkstoff mindestens eine Verbindung nach einem der Ansprüche 1 bis 4 in Kombination oder in Mischung mit einem inerten, nicht-toxischen, pharmazeutisch annehmbaren Bindemittel oder Trägermaterial.

6. Pharmazeutische Zubereitungen nach Anspruch 5, dadurch gekennzeichnet, daß das Bindemittel oder Trägermaterial für die parenterale oder orale Verabreichung geeignet ist.

7. Verfahren zur Herstellung der Verbindungen der allgemeinen Formel I nach Anspruch 1, dadurch gekennzeichnet, daß man

a) ein Lacton der allgemeinen Formel (III) des Formelschemas, in der die Substituenten X, Y und A die für die allgemeine Formel (I) angegebenen Bedeutungen besitzen, der selektiven Wirkung eines (Alkoxycarbonylmethyl)-phosphonium-ylids der Formel (IV)

$$Ar_3P^+—CH^-—COOR_4 \qquad (IV)$$

in der Ar für eine Phenylgruppe oder eine substituierte Phenylgruppe und $R_4$ für eine gegebenenfalls substituierte niedrigmolekulare Alkylgruppe oder Aralkylgruppe stehen, unterwirft zur Bildung des entsprechenden (Alkoxycarbonyl)-methylidenderivats der allgemeinen Formel (V) des Formelschemas 1, in der die Substituenten A, X, Y und $R_4$ die oben angegebenen Bedeutungen besitzen,

b) welches man der Einwirkung eines Hydrierungsmittels unterwirft zur Bildung eines (Alkoxycarbonyl)-methylenderivats der allgemeinen Formel (VI) des Formelschemas 1, worin die Substituenten X, Y, $R_4$ und A die oben angegebenen Bedeutungen besitzen,

c) oder welches man durch Einwirkung eines Hydrierungsmittels, insbesondere eines gemischten Alkalimetallhydrids reduziert zur Bildung eines Alkohols der allgemeinen Formel (VII) des Formelschemas 1, worin X, Y und A die oben angegebenen Bedeutungen besitzen,

d) welchen Alkohol man mit einer Halogenwasserstoffsäure oder einer Sulfonsäure in das entsprechende Halogenid oder Sulfonat der Formel (VIII) des Formelschemas 1 umwandelt, in der die Definition der Substituenten X, Y und A unverändert bleibt und Z Cl, Br, J, Alkyl-$SO_3$ oder Aryl-$SO_3$ (insbesondere das p-Toluolsulfonat) bedeutet, wonach man

e) diese Verbindung mit einem Amin der allgemeinen Formel (IX)

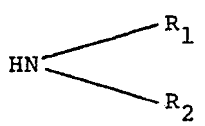
(IX)

in der $R_1$ und $R_2$ die bezüglich der Formel (I) angegebenen Bedeutungen besitzen umsetzt, so daß man ein Ethylaminderivat der allgemeinen Formel (I) erhält.

8. Verfahren zur Herstellung der Verbindungen der allgemeinen Formel (I) nach Anspruch 1, dadurch gekennzeichnet, daß man ein Lacton der allgemeinen Formel (III), in der X, Y und A die bezüglich der Formel (I) angegebenen Bedeutungen besitzen, der Einwirkung eines Cyanomethyl-phosphonium-ylids der allgemeinen Formel

$$Ar_3P^+—CH^-—CN \qquad (IV')$$

in der Ar für eine gegebenenfalls substituierte Phenylgruppe steht, unterwirft zur Bildung eines Cyanomethylidenderivats der allgemeinen Formel XIV des Formelschemas 2 worin X, Y und A die oben angegebenen Bedeutungen besitzen,

o) welches man selektiv zu den entsprechenden Aminoethylidenderivaten der allgemeinen Formel XV in der Form Z oder E hydriert, worin die Substituenten A, X und Y die oben angegebenen Bedeutungen besitzen, wonach man

p) die letzteren mit einem Hydrierungsmittel, wie einem Metallhydrid oder durch katalytische Hydrierung zu einem Ethylaminderivat der Formel (I) hydriert, in der $R_1$ und $R_2$ Wasserstoffatome darstellen, welches man gewünschtenfalls mit Verbindungen der Formel $R_1Z$ und/oder $R_2Z$, worin Z für

ein Halogenatom oder eine Sulfonylgruppe steht, alkylieren kann, so daß man die entsprechenden Verbindungen erhält, in denen $R_1$ und/oder $R_2$ für eine niedrigmolekulare Alkylgruppe stehen.

9. Verfahren zur Herstellung der Verbindungen der allgemeinen Formel (I) nach Anspruch 1, dadurch gekennzeichnet, daß man

q) eine Alkoxycarbonylmethylidenverbindung der allgemeinen Formel (V) einer milden Verseifung in alkalischem Medium unterwirft zur Bildung eines (Hydroxycarbonyl)-methylidenderivats der allgemeinen Formel (XVI) in der Form Z oder E des Formelschemas 3, worin die Substituenten X, Y und A die oben angegebenen Bedeutungen besitzen,

r) welches man in Gegenwart eines die Carboxylgruppe funktionalisierenden Mittels (Cyanurylchlorid oder Carbodiimid) mit einem Amin der oben definierten allgemeinen Formel (IX) zur Bildung des entsprechenden Methylidenamids der allgemeinen Formel XVI, in der die Substituenten $R_1$, $R_2$, X, Y und A die oben angegebenen Bedeutungen besitzen, kondensiert und

s) dieses zu dem Ethylaminderivat der allgemeinen Formel (I) reduziert.

**Ansprüche** (für den Vertragsstaat AT)

1. Verfahren zur Herstellung der Verbindungen der allgemeinen Formel

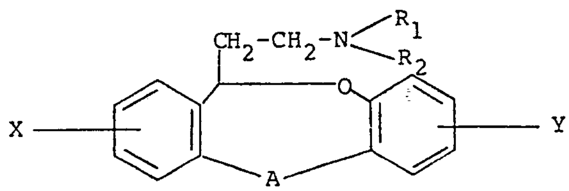

(I)

in der

X und Y, die gleichartig oder verschieden sein können, Wasserstoffatome oder Halogenatome, Alkylgruppen mit 1 bis 4 Kohlenstoffatomen, Alkoxygruppen mit bis 4 Kohlenstoffatomen oder Trifluormethylgruppen

A eine Methylengruppe oder eine Iminogruppe der Formel $—NR_3—$, worin $R_3$ für ein Wasserstoffatom oder eine Alkyl- oder Alkanoyl-Gruppe mit 1 bis Kohlenstoffatomen steht, und

$R_1$ und $R_2$, die gleichartig oder verschieden sein können, jeweils Wasserstoffatome oder niedrigmolekulare Alkylgruppen oder gemeinsam mit dem Stickstoffatom, an das sie gebunden sind, eine Pyrrolidinogruppe, eine Piperidinogruppe oder eine 4-Methyl-piperazinogruppe bedeuten,

in Form der Racemate oder der optischen Isomeren sowie ihrer Additionssalze mit einer anorganischen oder organischen therapeutisch verträglichen Säure, dadurch gekennzeichnet, daß man

a) ein Lacton der allgemeinen (III) des Formelschemas, in der die Substituenten X, Y und A die für die allgemeine Formel (I) angegebenen Bedeutungen besitzen, der selektiven Wirkung eines (Alkoxycarbonylmethyl)-phosphonium-ylids der Formel (IV)

$$Ar_3P^+—CH^-—COOR_4 \qquad (IV)$$

in der Ar für eine Phenylgruppe oder eine substituierte Phenylgruppe und $R_4$ für eine gegebenenfalls substituierte niedrigmolekulare Alkylgruppe oder Aralkylgruppe stehen, unterwirft zur Bildung des entsprechenden Alkoxycarbonyl-methylidenderivats der allgemeinen Formel (V) des Formelschemas 1, in der die Substituenten A, X, Y und $R_4$ die oben angegebenen Bedeutungen besitzen,

b) welches man der Einwirkung eines Hydrierungsmittels unterwirft zur Bildung eines (Alkoxycarbonyl)-methylenderivats der allgemeinen Formel (VI) des Formelschemas 1, worin die Substituenten X, Y, $R_4$ und A die oben angegebenen Bedeutungen besitzen,

c) oder welches man durch Einwirkung eines Hydrierungsmittels, insbesondere eines gemischten Alkalimetallhydrids reduziert zur Bildung eines Alkohols der allgemeinen Formel (VIII) des Formelschemas 1, worin X, Y und A die oben angegebenen Bedeutungen besitzen,

d) welchen Alkohol man mit einer Halogenwasserstoffsäure oder einer Sulfonsäure in das entsprechende Halogenid oder Sulfonat der Formel (VIII) des Formelschemas 1 umwandelt, in der die Definition der Substituenten X, Y und A unverändert bleibt und Z Cl, Br, J, $Alkyl-SO_3$ oder $Aryl-SO_3$ (insbesondere das p-Toluolsulfonat) bedeutet, wonach man

e) diese Verbindung mit einem Amin der allgemeinen Formel (IX)

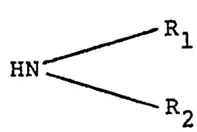

(IX)

in der $R_1$ und $R_2$ die bezüglich der Formel (I) angegebenen Bedeutungen besitzen umsetzt, so daß man ein Ethylaminderivat der allgemeinen Formel (I) erhält.

2. Verfahren zur Herstellung der Verbindungen der allgemeinen Formel (I) nach Anspruch 1, dadurch gekennzeichnet, daß man ein Lacton der allgemeinen Formel (III), in der X, Y und A die bezüglich der Formel (I) angegebenen Bedeutungen besitzen, der Einwirkung eines Cyanomethyl-phosphonium-ylids der allgemeinen Formel

$$Ar_3P^+—CH^-—CN \qquad (IV')$$

in der Ar für eine gegebenenfalls substituierte Phenylgruppe steht, unterwirft zur Bildung eines Cyanomethylidenderivats der allgemeinen Formel XIV des Formelschemas 2 worin X, Y und A die oben angegebenen Bedeutungen besitzen,

o) welches man selektiv zu den entsprechenden Aminoethylidenderivaten der allgemeinen Formel XV in der Form Z oder E hydriert, worin die Substituenten A, X und Y die oben angegebenen Bedeutungen besitzen, wonach man

p) die letzteren mit einem Hydrierungsmittel, wie einem Metallhydrid oder durch katalytische Hydrierung zu einem Ethylaminderivat der Formel (I) hydriert, in der $R_1$ und $R_2$ Wasserstoffatome darstellen, welches man gewünschtenfalls mit Verbindungen der Formel $R_1Z$ und/oder $R_2Z$, worin Z für ein Halogenatom oder eine Sulfonylgruppe steht, alkylieren kann, so daß man die entsprechenden Verbindungen erhält, in denen $R_1$ und/oder $R_2$ für eine niedrigmolekulare Alkylgruppe stehen.

3. Verfahren zur Herstellung der Verbindungen der allgemeinen Formel (I) nach Anspruch 1, dadurch gekennzeichnet, daß man

q) eine Alkoxycarbonylmethylidenverbindung der allgemeinen Formel (V) einer milden Verseifung in alkalischem Medium unterwirft zur Bildung eines (Hydroxycarbonyl)-methylidenderivats der allgemeinen Formel (XVI) in der Form Z oder E des Formelschemas 3, worin die Substituenten X, Y und A die oben angegebenen Bedeutungen besitzen,

r) welches man in Gegenwart eines die Carboxylgruppe funktionalisierenden Mittels (Cyanurylchlorid oder Carbodiimid) mit einem Amin der oben definierten allgemeinen Formel (IX) zur Bildung des entsprechenden Methylidenamids der allgemeinen Formel XVI, in der die Substituenten $R_1$, $R_2$, X, Y und A die oben angegebenen Bedeutungen besitzen, kondensiert und

s) dieses zu dem Ethylaminderivat der allgemeinen Formel (I) reduziert.

SCHEMA DE SYNTHESE

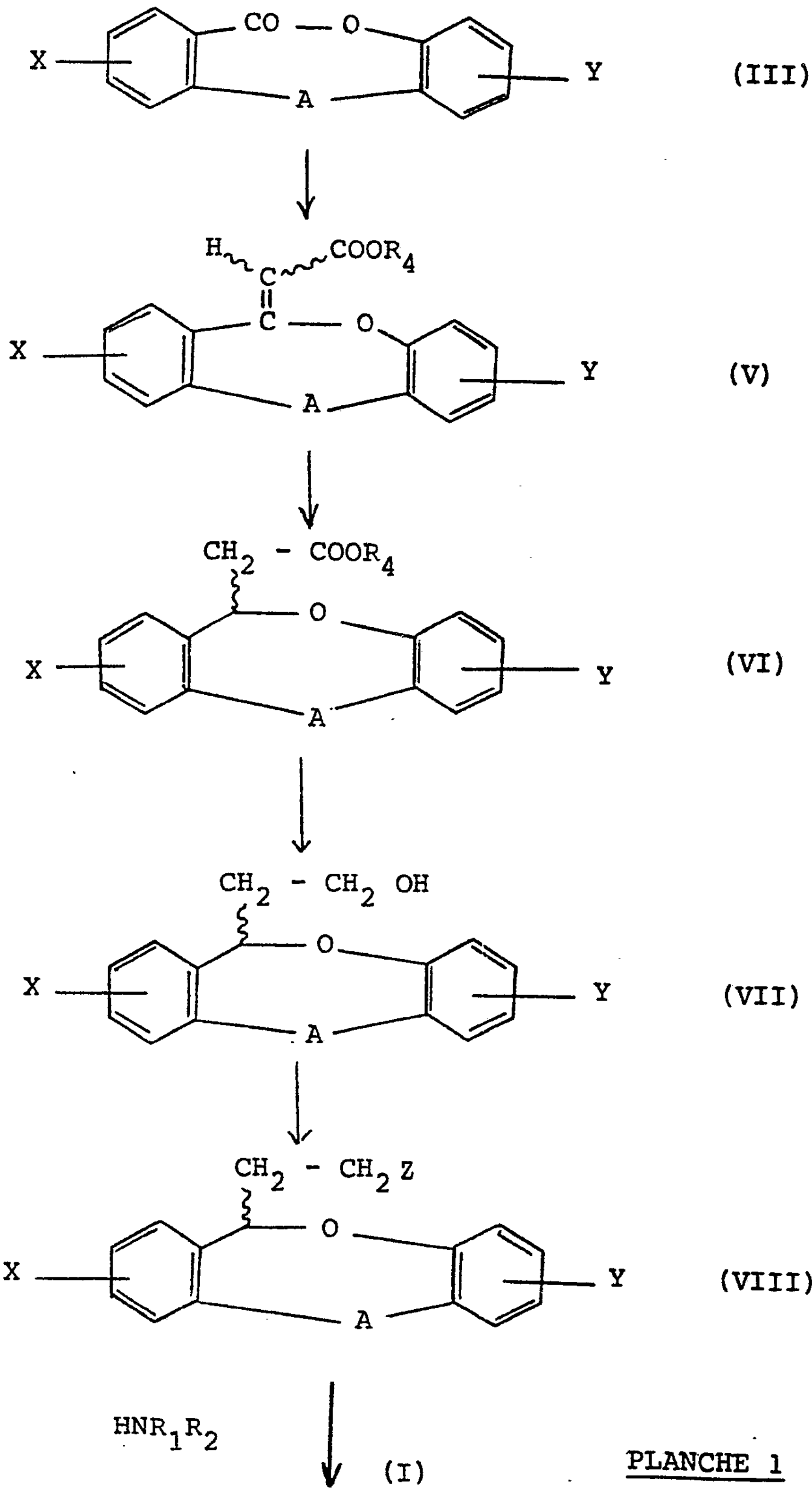


(III)

a)

(V)

b)

(VI)

c)

(VII)

d)

(VIII)

e)

PLANCHE 1

PLANCHE 2

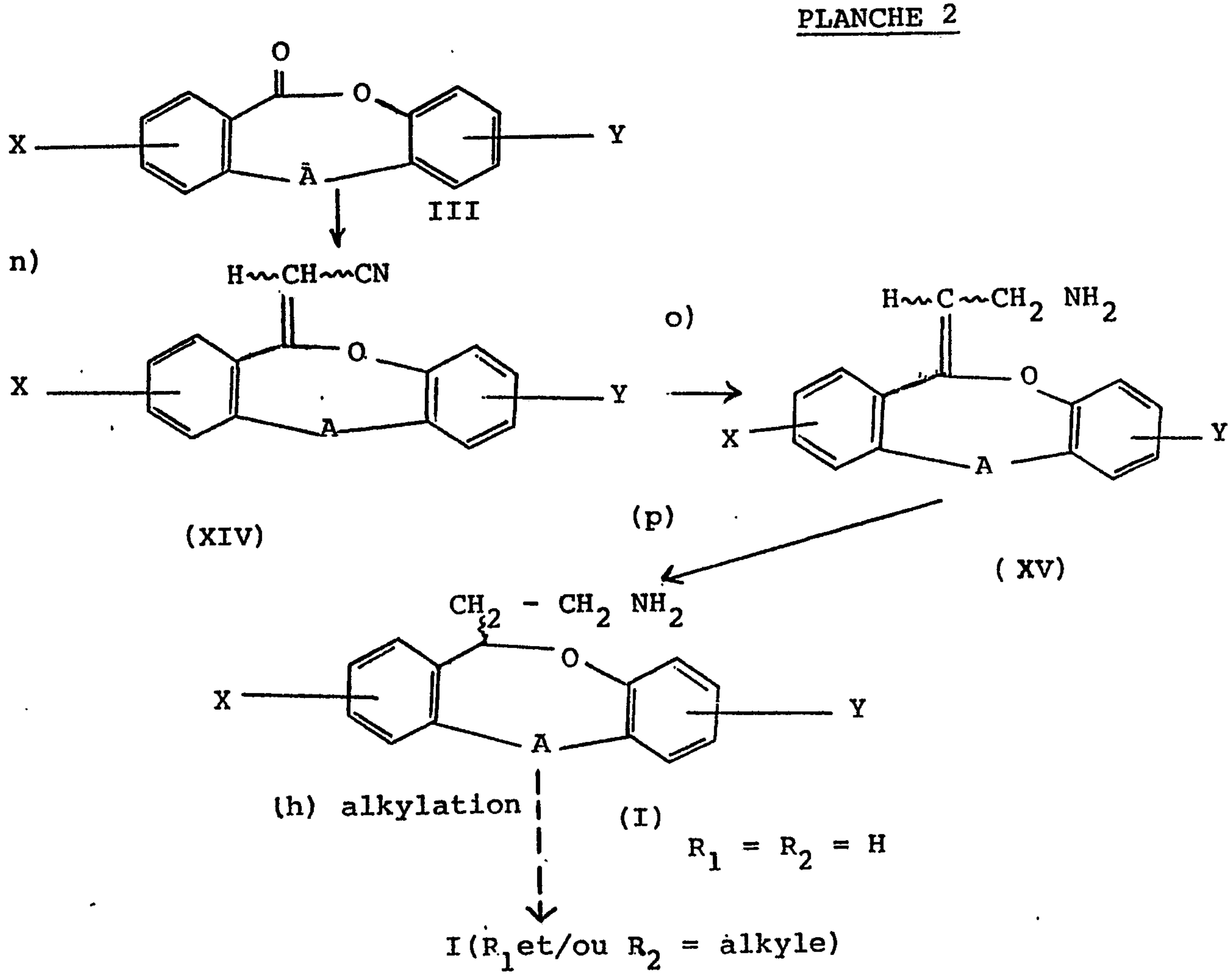

PLANCHE 3

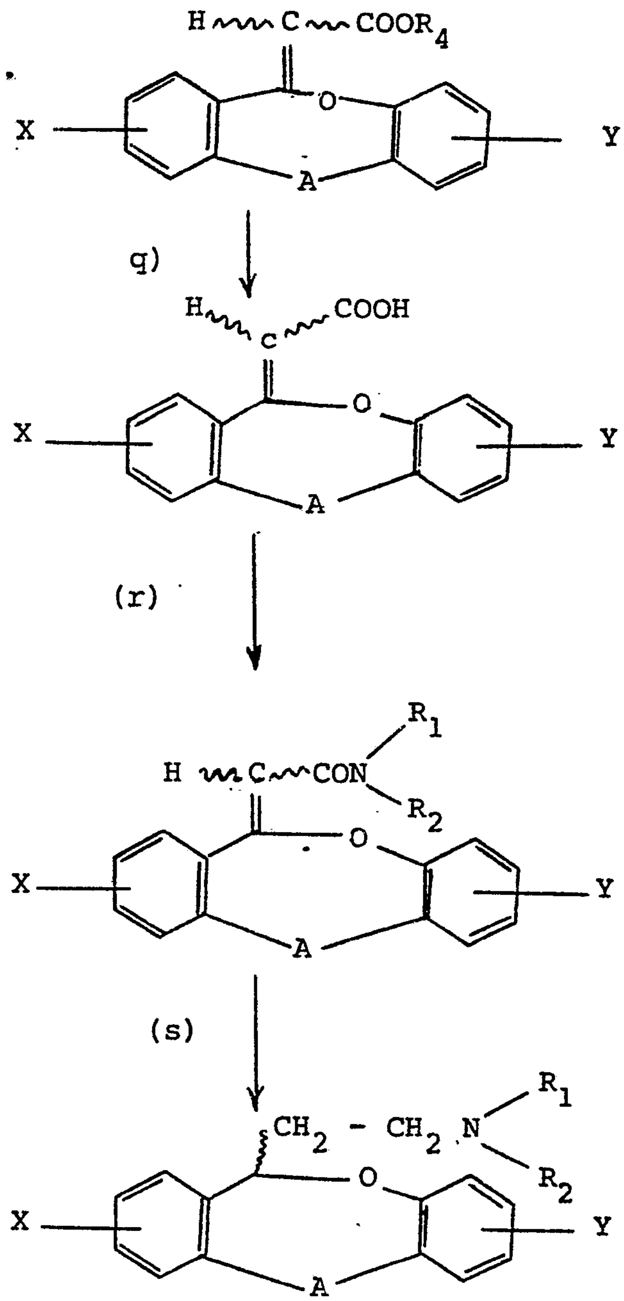


(V)

(XVI)

(XVII)

(I)